# EUROPEAN PATENT SPECIFICATION

(11) **EP 2 596 778 B1**
(45) Date of publication and mention of the grant of the patent: **21.03.2018**
(21) Application number: 12194051.4
(22) Date of filing: 23.11.2012
(51) Int. Cl.: A61F 13/15, B65H 35/00, B65H 35/08, B65H 39/14

(54) **Apparatus and method for applying short machine direction adhesive patches to a carrier layer**
Vorrichtung und Verfahren zum Aufbringen von Haftpflastern in kurzer Maschinenrichtung und Trägerschicht
Appareil et procédé pour appliquer des timbres adhésifs de sens de machine court à une couche de support

(30) Priority: 23.11.2011 US 201161563322 P
(43) Date of publication of application: 29.05.2013
(73) Proprietor: Curt G. Joa, Inc., Sheboygan Falls, Wisconsin 53085 (US)
(72) Inventor: Shin, Sang Hyub, Sheboygan, Wisconsin 53081 (US); Pelland, Jon Allen, Sheboygan, Wisconsin 53081 (US); Gutowski, Jesse, De Pere, Wisconsin 54115 (US); Veldman, Cory, Plymouth, Wisconsin 53073 (US); Jenquin, Peter J, Plymouth, Wisconsin 53073 (US); Kiela II, Gene F, Plymouth, Wisconsin 53073 (US); Nelson, Chris, Dallas, Georgia 30157 (US)
(74) Representative: Potter Clarkson LLP

(56) References cited:
- EP-A1- 0 411 287
- EP-A1- 0 676 352

## Description

### Background of the Invention

During production of disposable products such as diapers, adhesives are often used to couple different woven, non-woven, or elastic components together. Based on desired product configuration, adhesives are often desired to be applied to a web running in the machine direction. Often, it is desired to have this adhesive be applied to a very short distance in the machine direction to create a narrow adhesive patch in the machine direction, and relative wider in the cross machine direction.

This invention relates to the application of adhesives to moving webs. Adhesives can be used to couple discrete elements of disposable products onto a running continuous web of material.

For instance, some disposable products are formed of a continuous running web of material, such as a non-woven material, to which discrete components are applied. Often the discrete components are coupled to the running web of material by adhesive materials. It is preferable that the adhesive applied to the material be applied to the continuous running web in a pattern matching a footprint of the discrete component. Such patterns can be of any shape, such as typical geometric shapes, but are typically square or rectangular.

In automated machinery, there are processes that can limit top machine production speed. The application of hot melt adhesive on a running web is one such process. For example, conventional, commercially available hot melt adhesive application systems have a minimum valve open time of 4ms to 5ms. When applying an adhesive to a running web, the valve typically cannot open and close in times less than this 4ms to 5ms range. As web speed increases the minimum length of applied adhesive stripe increases. For example, if an adhesive pattern is applied by an applicator with a valve open time of 5ms on a web that is traveling at 500FPM, the same applicator would require a valve open time of 2.5ms to apply the same length adhesive stripe on a web traveling at 1000 FPM.

If only a very small portion (or length, in the machine direction) of adhesive is desired on a running web, present adhesive applicators limit the speed of the running web, because faster speed of the running web results in a longer, and perhaps too long, application of adhesive in the machine direction.

Generally, diapers comprise an absorbent insert or patch and a chassis, which, when the diaper is worn, supports the insert proximate a wearer's body. Additionally, diapers may include other various patches, such as tape tab patches, reusable fasteners and the like. The raw materials used in forming a representative insert are typically cellulose pulp, tissue paper, poly, nonwoven web, acquisition, and elastic, although application specific materials are sometimes utilized. Usually, most of the insert raw materials are provided in roll form, and unwound and applied in assembly line fashion.

In the creation of a diaper, multiple roll-fed web processes are typically utilized. To create an absorbent insert, the cellulose pulp is unwound from the provided raw material roll and pulverized by a pulp mill. Discrete pulp cores are formed by a core forming assembly and placed on a continuous tissue web. Optionally, super-absorbent powder may be added to the pulp core. The tissue web is wrapped around the pulp core. The wrapped core is debulked by proceeding through a calender unit, which at least partially compresses the core, thereby increasing its density and structural integrity. After debulking, the tissue-wrapped core is passed through a segregation or knife unit, where individual wrapped cores are cut. The cut cores are conveyed, at the proper pitch, or spacing, to a boundary compression unit.

While the insert cores are being formed, other insert components are being prepared to be presented to the boundary compression unit. For instance, the poly sheet is prepared to receive a cut core. Like the cellulose pulp, poly sheet material is usually provided in roll form. The poly sheet is fed through a splicer and accumulator, coated with an adhesive in a predetermined pattern, and then presented to the boundary compression unit. In addition to the poly sheet, which may form the bottom of the insert, a two-ply top sheet may also be formed in parallel to the core formation. Representative plies are an acquisition web material and a nonwoven web material, both of which are fed from material rolls, through a splicer and accumulator. The plies are coated with adhesive, adhered together, cut to size, and presented to the boundary compression unit. Therefore, at the boundary compression unit, three components are provided for assembly: the poly bottom sheet, the core, and the two-ply top sheet.

A representative boundary compression unit includes a die roller and a platen roller. When all three insert components are provided to the boundary compression unit, the nip of the rollers properly compresses the boundary of the insert. Thus, provided at the output of the boundary compression unit is a string of interconnected diaper inserts. The diaper inserts are then separated by an insert knife assembly and properly oriented. At this point, the completed insert is ready for placement on a diaper chassis.

A representative diaper chassis comprises nonwoven web material and support structure. The diaper support structure is generally elastic and may include leg elastic, waistband elastic and belly band elastic. The support structure is usually sandwiched between layers of the nonwoven web material, which is fed from material rolls, through splicers and accumulators. The chassis may also be provided with several patches, besides the absorbent insert. Representative patches include adhesive tape tabs and resealable closures.

The process utilizes two main carrier webs; a nonwoven web which forms an inner liner web, and an outer web that forms an outwardly facing layer in the finished diaper. In a representative chassis process, the nonwoven web is slit at a slitter station by rotary knives along three lines, thereby forming four webs. One of the lines is on approximately the centerline of the web and the other two lines are parallel to and spaced a short distance from the centerline. The effect of such slicing is twofold; first, to separate the nonwoven web into two inner diaper liners. One liner will become the inside of the front of the diaper, and the second liner will become the inside of the back of that garment. Second, two separate, relatively narrow strips are formed that may be subsequently used to cover and entrap portions of the leg-hole elastics. The strips can be separated physically by an angularly disposed spreader roll and aligned laterally with their downstream target positions on the inner edges of the formed liners.

After the nonwoven web is sliced, an adhesive is applied to the liners in a predetermined pattern in preparation to receive leg-hole elastic. The leg-hole elastic is applied to the liners and then covered with the narrow strips previously separated from the nonwoven web. Adhesive is applied to the outer web, which is then combined with the assembled inner webs having elastic thereon, thereby forming the diaper chassis. Next, after the elastic members have been sandwiched between the inner and outer webs, an adhesive is applied to the chassis. The chassis is now ready to receive an insert.

To assemble the final diaper product, the insert must be combined with the chassis. The placement of the insert onto the chassis occurs on a placement drum or at a patch applicator. The inserts are provided to the chassis on the placement drum at a desired pitch or spacing. The generally flat chassis/insert combination is then folded so that the inner webs face each other, and the combination is trimmed. A sealer bonds the webs at appropriate locations prior to individual diapers being cut from the folded and sealed webs.

Disposable diapers are susceptible to leakage of feces of a diaper wearer that escapes the diaper upwards in the lower back region of the wearer. To solve this problem, a fully adhered, cross-machine direction stretch waist band can be applied to the body side of the diaper's top sheet, or with a pocket feature, which requires a zone of non-adhesive in the center - forming a pocket.

The existing product is made using a process of creating a machine direction stretch laminate, extended and applied to a puck, and then cut, turned and placed onto the product at matched speed, such as taught in U.S. Patent No. 6,319, 347. A cross-machine direction stretch laminate (commercially available from manufacturers such as Aplix, Koester, Tredegar and the like) can be used, which may consist of a tri-laminate, with an elastomeric film in the center and nonwoven facings on both sides.

It is difficult however to provide the necessary intermittent adhesive application, provided in a short length in the machine direction and the cross-machine direction to a high speed (1200FPM +) carrier web.

One application of short length adhesive in the machine direction is used in applying waistband material. One side of the waist material is touched with a vacuum anvil roll (as well as infeed conveyor hardware), and that same piece of waist material - once cut - on the opposite side, is also touched in order to provide the cross-machine direction stretch. The cut and stretched piece is then applied to the running topsheet web. This process works well when a patch is desired to be placed on the interior side of that nonwoven topsheet web, but it is more difficult to apply the waist piece to the exterior, body side of the nonwoven topsheet.

One application of short length adhesive in the machine direction is used in order to provide a feces capturing structure. Adhesive required between a feces capturing structure and a topsheet assembly (including cuff in some cases) needs to be applied to the topsheet. Because the topsheet travels at a high rate of speed, and the intermittent portion of the adhesive pattern is very short (approx. 12mm), it is difficult to perform this application with an intermittent adhesive applicator, because a 12 mm adhesive pattern in the machine direction, which carries webs at speeds such as 1250 feet/minute, web results in an intermittent on time of 2 milliseconds, difficult to achieve.

It is desirable to provide disposable products with short adhesive patches carried by an intermediate carrier layer. In a preferred embodiment, the techniques are used to create a rear body, downward facing pocket in a waistband area of a diaper.

### Summary of the Invention

According to the present invention there is provided a method according to claim 1. In order to overcome processes that can limit top machine production speed, a novel application of hot melt adhesive on a running web is disclosed in order to provide a small portion (or length, in the machine direction) of adhesive on a running web.

In one embodiment of the present invention, an intermediate web is provided, which could serve as one of the layers of a tri-laminate, which receives an intermittent adhesive application, and is processed through a traditional cut slip applicator (which only requires handling of the web from one side) . The intermittent timing required when the web length (per product) is much more favorable to an intermittent adhesive applicator.

Such an applicator can provide reliable performance down to a minimum on or off time of 4-6 milliseconds. 12mm on a 40mm web running at 1000 PPM requires a minimum timing of 18 milliseconds (well within operating range). Once that layer is applied to the top running web, a continuous adhesive pattern is applied to that chassis web / receiving patch combination at full length. The receiving layer is nonwoven, and is glued down using the desired pattern - and the CD stretch laminate is glued fully to the intermediate patch, and a feces capturing structure is provided.

In an alternative method, not claimed, a "full size" rectangular adhesive pattern is applied to the running web. A smaller, intermediate patch is applied to this adhesive pattern to cover a portion of the adhesive pattern, and a cross-machine direction elastic component is provided, again to result in a feces capturing structure.

In a second alternative method, not claimed, adhesive patterns are provided on outboard edges of the topsheet, and then a stretched waistband patch is applied onto the adhesive pattern. After that step, a non-extensible, non-woven patch can be applied, with the use of a traditional slip/cut unit. This patch could be glued continuously, and this patch anchors the waistband patch already adhered to the top sheet along one edge, and effectively provides a loose pocket to create the feces capturing structure.

The present method enables faster machine process speeds beyond performance capability of adhesive system(s) themselves. Additionally, adhesives can be masked so as to not be apparent from the outside of articles employed on. Further, complex adhesive pattern or patterns, such as stars, circles, etc. can be used in conjunction with a similar shape of mask put on top of the applied adhesives. Masks can be formed by laser cutting, die cutting, slitting, or other cutting forms known in the art.

These methods can also be employed to attain short glue patterns on otherwise fast moving webs.

### Brief Description of the Drawings

Fig. 1 is top perspective view of a prior art disposable product with a rear waistband feature for rear feces containment;
Fig. 2 is a perspective view of a main web carrying a rear waistband feces containment structure;
Fig. 3A is a top view of a first running web containing two machine direction adhesive applications, and a plurality of cross-machine direction adhesive applications;
Fig. 3B is a top view of the first running web showing lines of separation to create individual rear waistband feces containment features;
Fig. 3C is a side schematic view of a slip/cut applicator used to cut lines of separation to create individual rear waistband feces containment features, and apply the features to a main web;
Fig. 3D is a top view of a main web carrying a rear waistband feces containment feature;
Fig. 3E is a top view of a main web carrying a rear waistband feces containment feature covered by an elasticized waistband feature;
Fig. 4A is a top view of an alternate method of forming a rear waistband feces containment feature, with an adhesive patch laid onto a main web;
Fig. 4B shows a patch material used to partially conceal a portion of the adhesive patch;
Fig. 4C shows a pocket forming material, laid atop the first patch material;
Fig. 4D shows the pocket forming material deployed atop the first patch material and the unconcealed portion of the adhesive patch to form a pocket style rear waistband feces containment feature;
Fig. 5A is a top view of a second alternate method of forming a rear waistband feces containment feature, with a first running web containing two machine direction adhesive applications;
Fig. 5B is a top view of the first running web showing lines of separation to create first portions of individual rear waistband feces containment features;
Fig. 5C shows first portions of individual rear waistband feces containment features deposited onto the main web;
Fig. 5D shows deployment of a second, adhesive backed patch portion, which is deployed atop the first portions of individual rear waistband feces containment features;
Fig. 5E shows the second, adhesive backed patch portion deployed atop the first portion of individual rear waistband feces containment features to create a completed pocket style rear waistband feces containment feature.

### Description of the Preferred Embodiment

Although the disclosure hereof is detailed and exact to enable those skilled in the art to practice the invention, the physical embodiments herein disclosed merely exemplify the invention which may be embodied in other specific structures. While the preferred embodiment has been described, the details may be changed without departing from the invention, which is defined by the claims.

It is noted that the present techniques and apparatus are described herein with respect to products such as diapers, but as previously mentioned, can be applied to a wide variety of processes in which discrete components are applied sequentially.

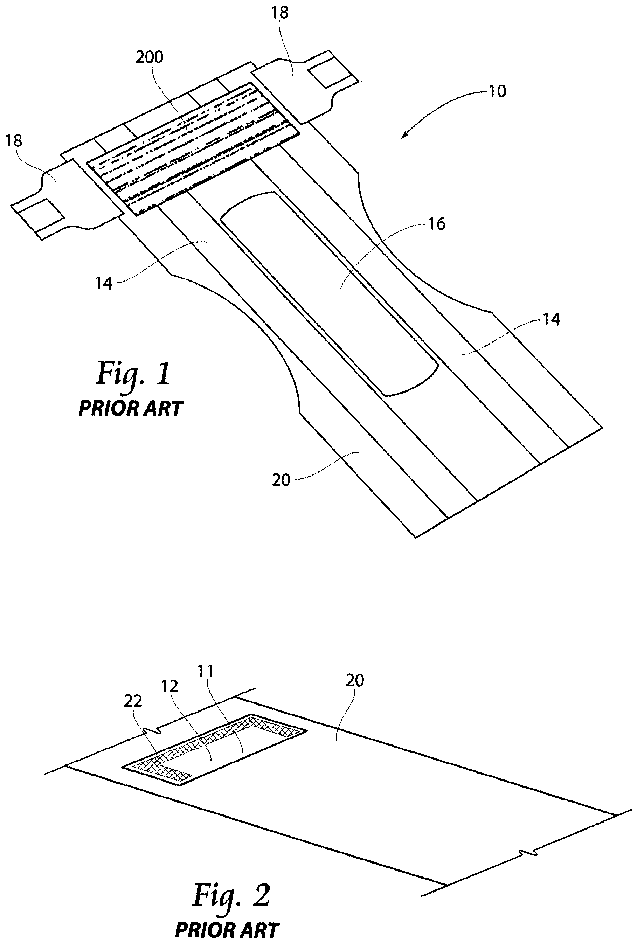

Referring now to Fig. 1 a top perspective view of a prior art disposable product 10 is shown, with a rear waistband feature 12 for rear feces containment. An inner nonwoven layer 20 is visible, with cuffs 14 provided in the side region of the diaper 10. An absorbent core is carried between the inner nonwoven layer and an outer layer (not shown). A combination ear/fastening mechanism 18 is provided to fasten the diaper 10 around the waist of a wearer.

The product of Fig. 1 can be made as either a fully adhered, cross-machine direction stretch waist band applied to the body side of the diaper' s top sheet, or with a "bowel movement dam" feature, which requires a zone of non-adhesive in the center - forming a pocket. The product of Fig. 1 is made using a process of creating a machine direction stretch laminate, extended and applied to a puck, and then cut, turned and placed onto the product at matched speed, as described in US 6,319,347.

Referring now to Fig. 2, a perspective view of a main web 20 carrying a rear waistband feces containment structure 11 is shown. This main web 20 is preferably the inner non-woven layer 20 of Fig. 1, and additional components of the diaper such as those shown in Fig. 1 (and others) can be combined with the main web 20 and rear waistband feces containment structure 11 in sequential fashion (not shown) as desired.

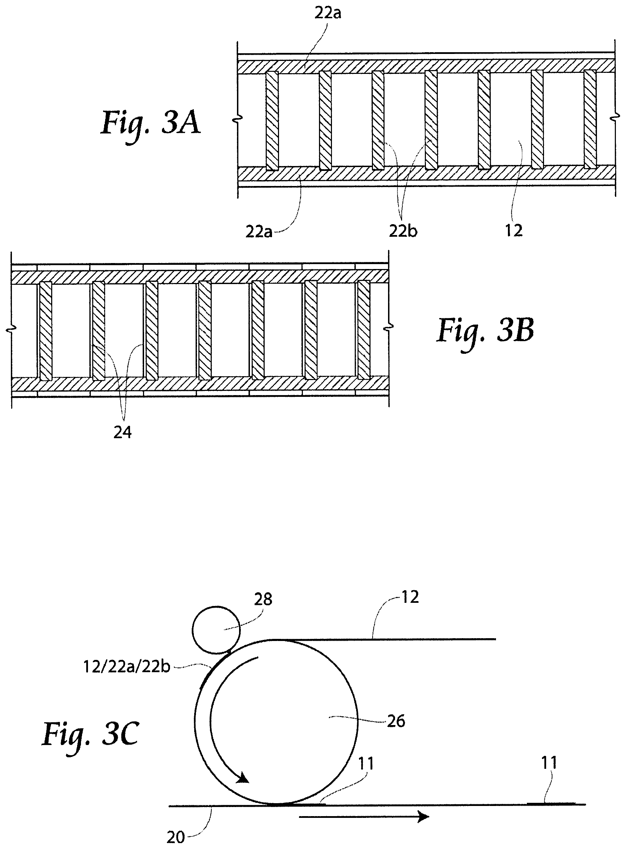

The present invention, shown generally in Figs. 3A - 3E relates to a method of forming the pocket-like rear waistband feces containment structure 11. The method can solve the problem of providing this product feature for use on disposable diapers to create a bowel movement dam feature, and can be used in other applications to attain short glue patterns on otherwise fast moving webs.

The embodiment, shown in Figs. 3a - 3E generally provides an intermediate web (which could serve as one of the layers of a tri-laminate) which receives an intermittent adhesive application, and is processed through a traditional cut slip applicator which only requires handling the web from one side, which is preferably a side not containing adhesive. The intermittent timing required when the web length (per product) is much slower, such as prior to a slip cut or accelerating unit, is more favorable to an intermittent adhesive applicator. Such an applicator can provide reliable performance down to a minimum on or off time of 4-6 milliseconds. 12mm on a 40mm web running at 1000 PPM requires a minimum timing of 18 milliseconds (well within operating range). Once that cross-machine direction adhesive layer is applied to the top running web, a continuous adhesive pattern is applied to that chassis web / receiving patch combination at full length. Because the receiving layer is nonwoven, and is glued down using the desired pattern - and the cross machine direction stretch laminate is glued fully to the intermediate patch, a short machine direction length of adhesive is provided.

Referring now to Fig. 3A, a top view of a first running web 12 containing two machine direction adhesive applications 22a is shown. A plurality of cross-machine direction adhesive applications 22b are also provided on the running web 12, which eventually forms the pocket-like rear waistband feces containment structure 11.

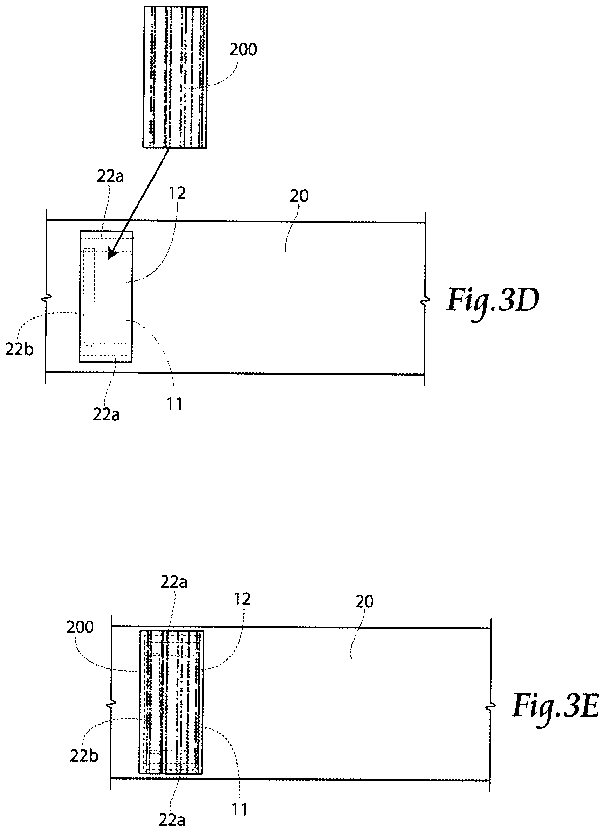

Referring now to Fig. 3B, first running web 12 is cut along lines of separation 24 to create individual rear waistband feces containment features by means such as a slip/cut applicator consisting of a vacuum anvil 26 and knife 28 combination as shown in Fig. 3C. The individual rear waistband feces containment features 11 are applied in such a fashion to an incoming inner non-woven web 20, resulting in the configuration shown in Fig. 3D. A pocket is formed by the downstream edges of the rear waistband feces containment features 11, which, because no adhesive is applied in that area, forms the pocket for containment of escaping matter from the rear of the diaper 10.

Referring now to Fig. 3E, the rear waistband feces containment feature 11 can be covered by an elasticized waistband feature 200, if desired, by slip cutting this material 200 onto the rear waistband feces containment feature 11 as previously described with relation to the slip cut unit shown in Fig. 3C.

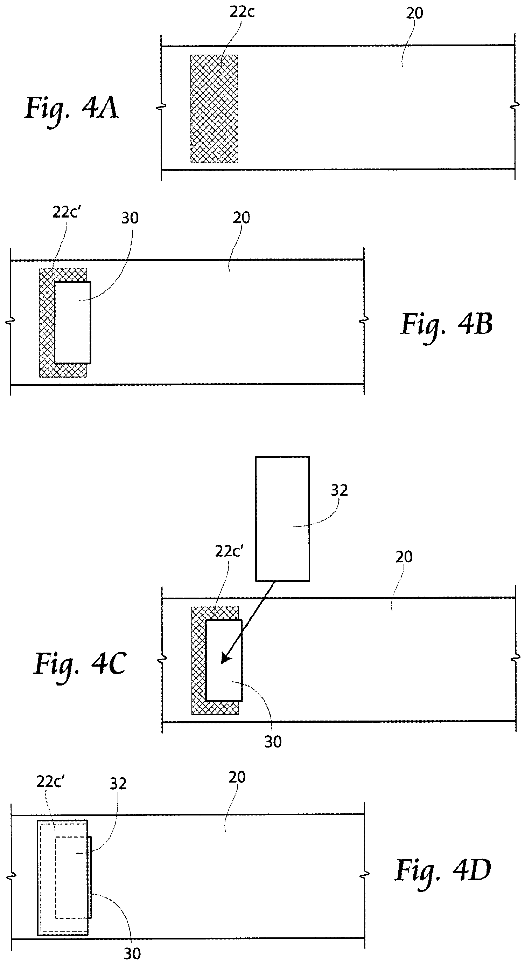

The second method, not claimed, described with reference to Figs. 4A-4D is generally to apply a "full size" rectangular adhesive pattern to the running web Placing a smaller, intermediate patch on this adhesive pattern and then applying the CD elastic waistpiece would be another approach to achieving a similar product performance result. Again, the adhesive applicator requirements here are not a challenge.

Referring now to Figs. 4A-4D, a top view of an alternate method, not claimed, of forming a rear waistband feces containment feature 11 is shown. In this method, an adhesive patch 22c is laid directly onto the main web 20, prior to receiving a piece of patch material 30, preferably slip/cut onto the main web 20. The patch material 30 covers a portion of the adhesive patch 22c, leaving an exposed portion of the adhesive patch 22c' as shown in Fig. 4B. The patch material serves as the bottom layer of the pocket structure. In this method, the inner non-woven can receive intermittently applied adhesive at an easily controlled interval.

Referring now to Fig. 4C, a pocket forming material 32 is laid atop the first patch material 30, and is coupled to the main web 20 at the exposed portion of the adhesive patch 22c' after being slip/cut and deposited again by a traditional slip/cut unit. In a preferred method, the pocket forming material 32 is a cross-machine direction stretch waistband. This configuration results in a pocket between the pocket forming material 32 and the first patch material 30, as shown in Fig. 4D to form a pocket style rear waistband feces containment feature 11.

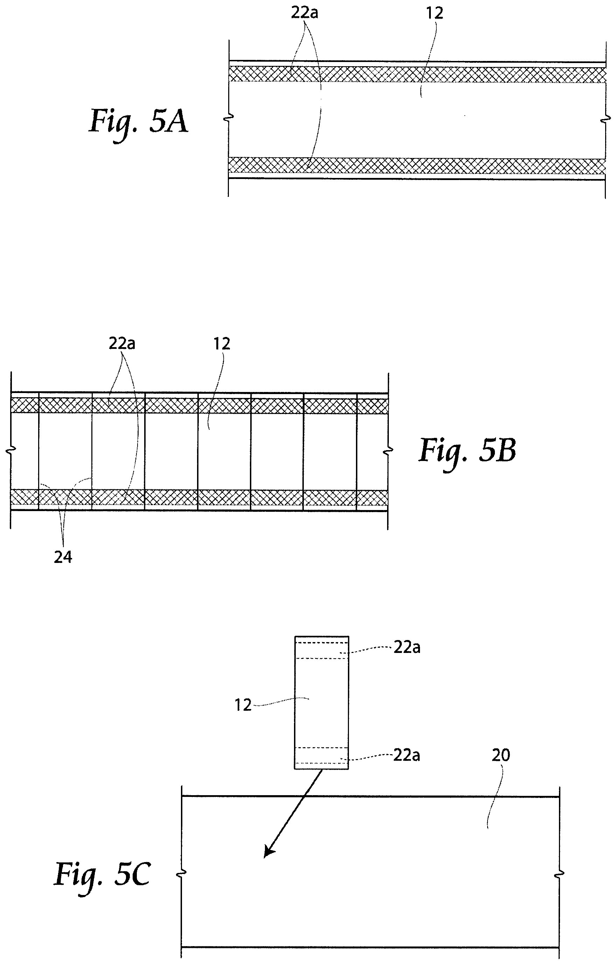

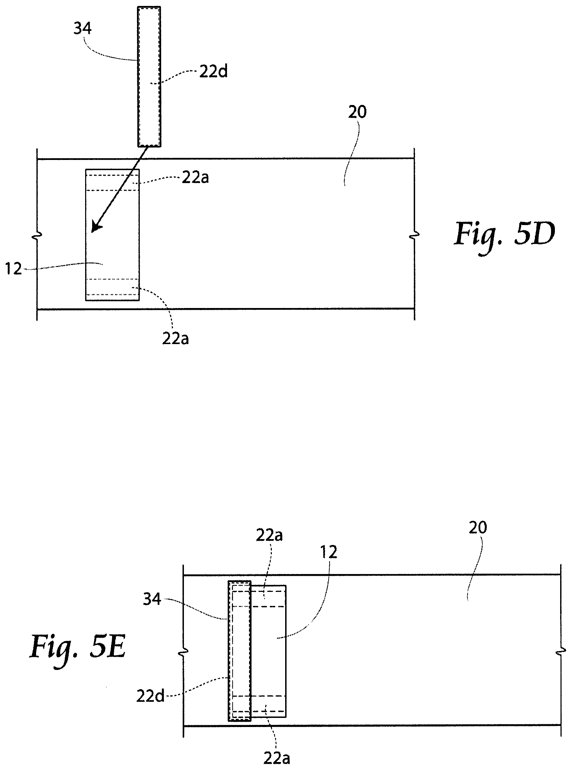

The third method, not claimed, described generally with reference to Figs. 5A - 5E is to provide adhesive patterns on the outboard edges of the topsheet, then applying the stretched waistband patch into the adhesive pattern. After that step, a non-extensible, NW patch could be applied (again, with the use of a traditional slip/cut unit). This patch could be glued continuously - with no fear of touching both sides of that web. Said patch would anchor the waistband patch already adhered to the top sheet - along one edge, and effectively providing that loose pocket for the BM dam function.

Referring now to Fig. 5A - 5E, top views of a second alternate method, not claimed, of forming a rear waistband feces containment feature 11 is shown. In this method a first running web 12 containing two machine direction adhesive applications 22 is slip/cut along lines 24 and deposited onto the main web 20 as shown in Figs. 5B and 5C. In a preferred method, the first running web 12 is a cross-machine direction stretch waistband.

Next, as shown in Fig. 5D, a second, adhesive backed patch portion 34 (preferably a non-woven material) or cross machine direction back pocket patch 34 is slip/cut onto the main web 20 and onto the rear of the previously deposited portions of the web 12. A small portion of the cross machine direction back pocket patch 34 extends rearward of the deposited portions of the web 12 thereby blocking the back end of the pocket formed underneath the deposited portions of the web 12 to provide a completed pocket style rear waistband feces containment feature 11.

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is defined by the claims.

## Claims

1. A method of providing a short machine direction glue pattern on a fast moving web, the method comprising:
providing a first intermediate web (12) at a first speed;
providing a top running web (20) running at a second speed which is faster than said first speed;
applying a plurality of cross machine direction adhesive patterns 22b onto said first intermediate web;
applying a machine direction adhesive pattern (22a) onto said first intermediate web;
processing said first intermediate web through a cut slip applicator (26, 28);
severing said first intermediate web between successive cross machine direction adhesive patterns to form discrete pieces;
accelerating said discrete pieces from said first speed to said second speed;
coupling said discrete pieces to said top running web around less than a periphery of said discrete pieces to form a pocket (11).

## Patentansprüche

1. Verfahren zum Bereitstellen eines Klebermusters in kurzer Maschinenrichtung auf einer sich schnell bewegenden Bahn, wobei das Verfahren Folgendes umfasst:
Bereitstellen einer ersten Zwischenbahn (12) mit einer ersten Geschwindigkeit;
Bereitstellen einer oben laufenden Bahn (20), die mit einer zweiten Geschwindigkeit, die schneller als die erste Geschwindigkeit ist, läuft;
Auftragen mehrerer Klebermuster (22b) in Maschinenquerrichtung auf die erste Zwischenbahn;
Auftragen eines Klebermusters (22a) in Maschinenrichtung auf die erste Zwischenbahn;
Verarbeiten der ersten Zwischenbahn durch einen Schnitt-Gleit-Applikator (26, 28);
Trennen der ersten Zwischenbahn zwischen aufeinanderfolgenden Klebermustern in Maschinenquerrichtung zum Ausbilden separater Teile;
Beschleunigen der separaten Teile von der ersten Geschwindigkeit auf die zweite Geschwindigkeit;
Verbinden der separaten Teile mit der oben laufenden Bahn um weniger als einen Umfang der separaten Teile herum zum Ausbilden einer Tasche (11).

## Revendications

1. Procédé de fourniture d'un court motif de colle sens machine sur une bande à déplacement rapide, le procédé comprenant :
la fourniture d'une première bande intermédiaire (12) à une première vitesse ;
la fourniture d'une bande en défilement supérieur (20) défilant à une seconde vitesse qui est plus rapide que ladite première vitesse ;
l'application d'une pluralité de motifs d'adhésif sens travers (22b) sur ladite première bande intermédiaire ;
l'application d'un motif d'adhésif sens machine (22a) sur ladite première bande intermédiaire ;
le traitement de ladite première bande intermédiaire au moyen d'un applicateur de coupe-glissement (26, 28) ;
le sectionnement de ladite première bande intermédiaire entre des motifs d'adhésif sens travers successifs pour former des pièces distinctes ;
l'accélération desdites pièces distinctes de ladite première vitesse à ladite seconde vitesse ;
le couplage desdites pièces distinctes sur ladite bande en défilement supérieur autour de moins d'une périphérie desdites pièces distinctes pour former une poche (11).
